# EUROPEAN PATENT SPECIFICATION

(11) **EP 2 059 223 B1**
(45) Date of publication and mention of the grant of the patent: **24.10.2012**
(21) Application number: 07802130.0
(22) Date of filing: 05.09.2007
(51) Int. Cl.: A61K 8/31, A61K 8/34, A61K 8/36, A61K 8/37, A61K 8/49, A61K 8/67, A61Q 19/08

(54) **SKIN CARE COMPOSITION**
HAUTPFLEGEZUSAMMENSETZUNG
COMPOSITION DE SOIN DE LA PEAU

(30) Priority: 08.09.2006 EP 06018843; 20.12.2006 EP 06026413
(43) Date of publication of application: 20.05.2009
(73) Proprietor: DSM IP Assets B.V., 6411 TE Heerlen (NL)
(72) Inventor: GORALCZYK, Regina, 79639 Grenzach-Wyhlen (DE); KLOCK, Jochen, 79104 Freiburg (DE); SCHWEIKERT, Loni, CH-4315 Zuzgen (CH)
(74) Representative: Schwander, Kuno
(86) International application number: PCT/EP2007/007720
(87) International publication number: WO 2008/028632

(56) References cited:
- WO-A-2004/105517
- WO-A-2006/056293
- FR-A- 2 694 692
- US-A1- 2004 258 645
- DATABASE WPI Week 200113 Derwent Publications Ltd., London, GB; AN 2001-121704 XP002465876 & RO 437 063 A (ANONYMOUS) 10 September 2000 (2000-09-10)

## Description

The present invention relates to an oral composition containing an optimal combination of nutritional ingredients at high concentrations and at optimal ratios in order to maintain and/or achieve health and/or a beautiful appearance of the human skin.

The skin is the largest organ of the human body. It is made up of multiple layers of epithelial tissues that guard underlying muscles and organs. As the interface with the surroundings, it plays the most important role in protecting the body against pathogens. It furthermore provides sensory perception, moisture control, shape, regulation of metabolic processes, immune response and insulation against heat and cold. The skin also has the remarkable role of defining our individuality by transmitting information back to the environment about a person's appearance, behavior and health.

The outermost layer of the skin is called epidermis. It forms the waterproof, protective wrap over the body's surface and is made up of stratified squamous epithelium with an underlying basal lamina. It contains no blood vessels, and is nourished by diffusion from the dermis. The main types of cells which make up the epidermis are keratinocytes, with melanocytes and Langerhans cells also present. Epidermis is divided into several layers where cells are formed through mitosis at the innermost layers. They move up the strata changing shape and composition as they differentiate and become filled with keratin. They eventually reach the top layer called stratum corneum and become sloughed off, or desquamated. This process is called keratinization and takes place within weeks. The outermost layer of Epidermis consists of 25 to 30 layers of dead cells. Every day thousands of new skin cells are generated.

The body naturally looses water by constant gentle evaporation through the skin (transepidermal water loss, TEWL). Preventing excessive water loss is exceptionally important in itself - both to the skin itself and to the body as a whole. In the normal epidermis the water content gets less the closer we get to the surface. Water makes up to 70 to 75 % of the weight of the basal layer, but only 10 to 15wt.-% of the stratum corneum. The presence of an adequate amount of water in the epidermis is important for the general appearance of a soft, smooth and attractive skin.

The stratum corneum is hydrated by the water trapped in lower layers as well as by normal perspiration. If it is deficient in natural moisturising factors or subjected to extreme weather conditions, the skin loses moisture. It becomes dry and taut. As it becomes less supple, its protective function is reduced. The skin chaps, transepidermal water loss increases, and its barrier function is impaired. As the skin ages, sebum (or oil) production slows down. Production of moisture-binding substances also decreases. The skin becomes drier and less able to retain moisture. It assumes a flat, dull appearance and is less smooth. Fine lines and wrinkles begin to form and the skin is less firm.

There is a multitude of different intrinsic and external factors that influence skin condition and foster skin aging. Intrinsic factors are genetic predisposition, the normal aging process, or hormonal status. UV radiation, air pollution, smoking and/or allergenic compounds are external factors which can account for premature skin aging.
As the skin ages chronically it particularly loses elasticity. Chronic exposure to UV radiation (UVB and UVA) leads to premature skin aging through epidermal and dermal damage by oxidative stress and sunburn. Sunburn is the inflammatory reaction of the skin in response to excessive exposure to natural or artificial solar light of the UVB wavelength.
Hyperkeratosis, keratinocyte dysplasia and/or dermal elastosis occur in affected skin areas, clinically presenting as photoaged skin with actinic or solar keratosis. These precancerous lesions show an increased risk for the development of squamous cell carcinoma (SCC). The clinical condition of premature skin aging is accompanied with hyperpigmentation and dilated and twisted microvasculature, i.e. teleangiectasia.

In addition to other influencing factors also nutrition has a strong impact on the skin's condition. Optimization of the diet improves general skin condition and hydration of the skin. A deficiency in essential vitamins and/or fatty acids e.g. has clear cutaneous effects. Therefore for full skin protection, a well-balanced mix of different nutritional substances is essential. Optimally, micronutrients, i.e. vitamins, certain polyunsaturated fatty acids (PUFAs) and other nutritional active compounds such as antioxidants are supported internally, i.e. by oral administration, absorption, and transport to the skin via the blood stream.

PUFAs are known to be important for the preservation of the skin-barrier function and the water content of the skin. They are incorporated into the cell membranes of skin keratinocytes and fibroblasts. Due to their structure, PUFAs enhance membrane fluidity, and thus contribute to more flexible and mobile cell membranes than saturated fatty acids. Oral intake of certain PUFAs such as gamma-linolenic acid or linoleic acid leads to a more moistened and smoother skin.

WO2006/056293 discloses oral compositions for enhancing skin appearance containing polyunsaturated fatty acids (DHA and EPA) and carotenoids in the range of 0.005 wt.% to 0.1 wt.%. WO2004/105517 discloses that ethylesters of PUFAs can be used in oral compositions as replacement for PUFAs.

US 2004/258645 discloses a kit comprising an oral composition containing PUFAs and carotenoids and a topical skin care composition. However, the oral composition contains very low amounts of carotenoids (0.0005 % to 0.1 %.

The present invention relates to an oral composition providing an optimal combination of nutritional ingredients at high concentrations and at optimum ratios, preferably in capsules whereas the composition should show excellent bioavailability.

The present invention relates to a composition for oral intake comprising
i) at least one component selected from the group consisting of polyunsaturated fatty acids and esters of polyunsaturated fatty acids - e.g. ethyl esters, mono-, di- and triglyceridesters; and
ii) at least one component selected from the group consisting of carotenoids, wherein the amount of carotenoids is in the range of 25 to 80 % by weight, based on the total weight of the composition.

According to the present invention the amount of the component(s) ii) is preferably in the range of 30 to 75 % by weight, more preferably in the range of 40 to 60 % by weight, based on the total weight of the composition.

Polyunsaturated fatty acids, which are suitable according to the present invention, are mono- or polyunsaturated carboxylic acids having preferably 16 to 24 carbon atoms and, in particular, 1 to 6 double bonds, particularly preferably having 4 or 5 or 6 double bonds.

The unsaturated fatty acids can belong both to the n-6 series and to the n-3 series. Polyunsaturated fatty acids of the n-3 series are preferred. Preferred examples of n-3 polyunsaturated acids are eicosapenta-5,8,11,14,17-enoic acid (EPA) and docosahexa-4,7,10,13,16,19-enoic acid (DHA), as well as arachidonic acid (ARA).

Preferred derivatives of the polyunsaturated fatty acids are their esters, for example glycerides and, in particular, triglycerides; particularly preferably the ethyl esters. Triglycerides of n-3 polyunsaturated fatty acids are especially preferred.

The triglycerides can contain 3 uniform unsaturated fatty acids or 2 or 3 different unsaturated fatty acids. They may also partly contain saturated fatty acids.

When the derivatives are triglycerides, normally three different n-3 polyunsaturated fatty acids are esterified with glycerin. In one preferred embodiment of the present invention triglycerides are used, whereby 30 % of the fatty acid part are n-3 fatty acids and of these 25 % are long-chain polyunsaturated fatty acids. In a further preferred embodiment commercially available ROPUFA® '30' n-3 Food Oil (DSM Nutritional Products Ltd, Kaiseraugst, Switzerland) is used.
In another preferred embodiment of the present invention the PUFA ester is ROPUFA® '75' n-3 EE. ROPUFA '75' n-3 EE is refined marine oil in form of an ethyl ester with minimum content of 72 % n-3 fatty acid ethyl ester. It is stabilized with mixed tocopherols, ascorbyl palmitate, citric acid and contains rosemary extract.

According to the present invention it can be advantageous to use naturally occurring oils (one ore more components) containing triglycerides of polyunsaturated fatty acids, for example marine oils (fish oils) and/or plant oils.

Preferred oils which comprise triglycerides of polyunsaturated fatty acids are olive oil, sunflower seed oil, evening primrose seed oil, borage oil, grape seed oil, soybean oil, groundnut oil, wheat germ oil, pumpkin seed oil, walnut oil, sesame seed oil, rapeseed oil (canola), blackcurrant seed oil, kiwifruit seed oil, oil from specific fungi and fish oils.

Alternatively other polyunsaturated fatty acids (e. g. omega-3 fatty acids; omega-6 fatty acids) and/or their derivatives may be used.

In a preferred embodiment of the present invention the carotenoids (one or more components) are selected from the groups of xanthophylls and carotenes, preferably from beta-carotene, lycopene, lutein, zeaxanthin, beta-cryptoxanthin, astaxanthin, and/or their esters;

The composition according to the present invention is most suitable to support healthy skin appearance and beauty.
In particular it
- supports skin nourishment from inside the body via the blood stream (systemically);
- supports a clear, pure appearance of the skin
- fosters hydration and moisturizes the skin;
- supports skin barrier function;
- provides protection against oxidative stress;
   (especially if the carotenoids are selected from the group consisting of beta-• carotene, lycopene);
- provides protection against UV-radiation
   (especially if the carotenoid is beta-carotene and/or lycopene)
- has a general anti-aging effect.

Thus, the composition according to the present invention supports beauty from inside. The composition also promotes skin repair and regeneration upon healing of injuries as well as the physiological renewal process.

Overall the composition according to the present invention is promoting an optimal health, a natural radiance and glow and/or a beautiful look of the skin. According to the present invention the term "beautiful look of the skin" comprises a good blood supply generating a natural pink tone, a natural radiance and glow, good elasticity, hydration and moisture of the skin, an intact skin barrier function and a pure, clear skin, i.e. a skin lacking major impurities and normal sebum secretion.

There are different possibilities to achieve a clear, pure skin; applying cosmetic products is the most common one. However, it is traditionally also known that a healthy diet can contribute further to a clear, pure skin.

"Impure" skin can be caused by various factors: skin irritation through various exogenous and endogenous stimuli (e.g. sun, wind, cold, dryness, incompatibility or sensitivity to crèmes, or other skin care products, overproduction of sebum consequently leading to pimples, black spots and acne). It is generally accepted that inflammatory processes in the epidermis are involved in the processes leading to skin irritation, sensitive skin and impure skin. It is furthermore well established, that in these mechanisms cytokines play an important role. Pro-inflammatory cytokines are released from keratinocytes in the epidermis upon inflammatory stimuli (Boniface K, Lecron JC, Bernard FX, Dagregorio G, Guillet G, Nau F, Morel F. Keratinocytes as targets for interleukin-10-related cytokines: a putative role in the pathogenesis of psoriasis, Eur. Cytokine Netw. 2005 Dec;16(4):309-19).
Accordingly, reduction of the response of skin cells to inflammatory stimuli can lead to an improvement of an impure skin condition and may help to produce a pure skin with healthy and natural radiance and glow.

It was not to be forseen by the person skilled in the art that a composition according to the present invention with an ideal composition consisting of PUFAs and/or their esters and carotenoids, water-soluble vitamins, fat-soluble vitamins, ubichinones and/or polyphenols in an optimum ratio to each other would support a pure, clear skin. It was especially surprising that the dietary, oral application of a composition according to the present invention will lead to a more sustainable effect of a clear skin appearance.

Optimal skin moisture and hydration means little transepidermal water loss and intact skin barrier function. Well-hydrated skin is more firm and toned. Hydration minimizes the appearance of fine lines and wrinkles.
The hydration level of the outermost skin layer (10 - 20 µm depth), the stratum corneum, may be measured with a corneometer. The corneometric measurement allows an interpretation about the condition of the skin, the skin type and the effects of pharmaceutical and cosmetic products.
The principle of the corneometer is based on a capacitance measurement of a dielectric medium. Water has a relatively high dielectric constant (D = 80) in contrast to most other substances (D < 7). Therefore even slightest changes in the hydratation of the skin's surface alter the dielectric constant and accordingly the capacitance of a precision measuring capacitor. A spring in the probe head ensures constant pressure of 3,5 N on the skin. The measuring surface is 49 mm². The reproducibilty and accuracy (± 3%) of the measurement is high and the measurement time is 1 s only (prevents occlusion effects).
Boosting of moisture-binding capability of skin enables it to retain its healthy glow. Super hydrated skin is softer to the touch and has a smoother appearance.

In the event of an injury that damages the skin's protective barrier, the body triggers a response called inflammation, which sends fluids carrying phagocytic white blood cells to the injury site. Once the invading microorganisms have been brought under control, the skin proceeds to heal itself. The ability of the skin to heal even after considerable damage has occurred is due to the presence of stem cells in the dermis and cells in the stratum basale of the epidermis, all of which can generate new tissue.
In healthy skin, the immune defense is intact, inflammation within physiological range and the self healing process functional. The composition as defined above can support skin repair e.g. by occlusion, by partly replacing the lipids or by triggering cellular lipid production.
Epidermal keratinocytes are produced constantly from stem cells in the basal layer of the epidermis. During their migration upwards, the keratinocytes run through a differentiation process. The result of epidermal differentiation is the formation of the stratum corneum, which consists of terminally differentiated, cornified keratinocytes. The process from proliferation to desquamation takes about one month. The composition as defined above supports this renewal process.

### Lycopene and derivatives thereof

The term "lycopene" as used herein includes all-E and Z-stereoisomers. Alternatively a tomato extract which contains high amounts of lycopene can also be used.

### Lutein and derivatives thereof

The term "lutein" as used herein includes all-E and Z-stereoisomers. Suitable derivatives are e.g. its mono-and di-esters, preferably esters of saturated alkanoic acids such as acetic, propionic, laurinic, myristinic, palmitic, stearic and succinic acid, esters of mono-unsaturated fatty acids such as oleic acid, and poly-unsaturated fatty acids such as linolic, linoleic, pentaenoic, docosahexaenoic and arachidonic acid, and mixtures thereof.

### Zeaxanthin and derivatives thereof

The term "zeaxanthin" as used herein includes all-E and Z-stereoisomers. Suitable derivatives are e.g. its mono-and di-esters, preferably esters of saturated alkanoic acids such as acetic, propionic, laurinic, myristinic, palmitic, stearic and succinic acid, esters of mono-unsaturated fatty acids such as oleic acid, and poly-unsaturated fatty acids such as linolic, linoleic, pentaenoic, docosahexaenoic and arachidonic acid, and mixtures thereof.

According to the present invention it is advantageous to administer the active ingredients in a way that their effective daily amounts ("daily dosages") are in the ranges given below. It is thereby irrelevant if the daily dosage is applied all at once (by a single dosage) or in multiple dosages.

PUFA(s), in particular n-3 polyunsaturated fatty acids and/or its derivatives (especially triglycerides): daily dosage for humans (70 kg person): from 50 mg to 8 g, preferred daily dosage for humans (70 kg person) from 300 mg to 2000 mg.

β-Carotene: daily dosage for humans (70 kg person): 0.1 to 50 mg, preferred daily dosage for humans (70 kg person): 1 and 30 mg, more preferred daily dosage for humans (70 kg person): 2 to 7 mg.

Lycopene: For usual applications the daily dosage for humans (usually determined for a 70 kg person) for lycopene should not exceed 40 mg, preferably not exceed 25 mg. In some embodiments of the invention the daily dosage for humans (70 kg person) for lycopene can be between 0.1 to 40 mg, more preferably between 0 5 to 25 mg.

Lutein: For usual applications the daily dosage for humans (usually determined for a 70 kg person) for lutein not exceed 60 mg, preferably not exceed 30 mg.
In some embodiments of the invention the daily dosage for humans (70 kg person) for lutein is between 0.1 to 60 mg, more preferably between 1.0 to 30 mg.

Zeaxanthin: daily dosage for humans (70 kg person): 0.1 to 20 mg, preferred daily dosage for humans (70 kg person): 2 to 7 mg, more preferred daily dosage for humans (70 kg person): ca. 4 mg.

If instead of 'pure active ingredients' an active ingredient comprising extract and/or oil is used, the amount of the extract and/or oil to be used may be derived from the concentration of the 'pure active ingredient' within the extract and/or oil and the finding of the optimal dosage is a matter of routine experimentation for the person skilled in the art.

In all embodiments of the present invention the definitions and the preferred daily dosages for the active ingredients as described above apply.

A composition for consumption by humans can comprise
a) at least one component selected from the group consisting of polyunsaturated fatty acids and esters of polyunsaturated fatty acids such as ethyl esters, mono-, di- and triglyceridesters, in amount in the range of from 0.50 mg to 120 mg per kg bodyweight of said humans, preferably from 5 mg to 30 mg per kg bodyweight of said humans; and at least one of the components b) to m),
b) β-carotene, in an amount in the range of from 0.0014 to 0.7 mg per kg bodyweight, preferably from 0.01 and 0.5 mg per kg bodyweight of said humans;
c) lycopene, preferably in an amount in the range of from 0.0014 to 0.6 mg, more preferably between 0.007 to 0.35 mg per kg bodyweight of said humans;
d) lutein, in an amount in the range of from 0.0014 to 0.86 mg, preferably between 0.014 to 0.5 mg per kg bodyweight of said humans;
e) zeaxanthin, in an amount in the range of from 0.0014 to 0.28 mg, preferably between 0.028 to 0.1 mg per kg bodyweight of said humans;
wherein the amount of the component(s) b) to e) is in the range of 25 to 80 % by weight, based on the total weight of the composition.

The amount of the component(s) b) to e) is preferably in the range of 30 to 75 % by weight, more preferably in the range of 40 to 60 % by weight, based on the total weight of the composition.

The invention relates to the use of the compositions according to claim 1 for supporting a healthy skin appearance and beauty, for supporting skin nourishment from inside the body via the blood stream (systemically), for supporting a clear, pure appearance of the skin, for fostering hydration, for moisturizing the skin, for supporting the skin barrier function, for providing protection against oxidative stress, for providing protection against UV-radiation and/or for providing a general anti-aging effect, in particular for promoting skin repair a natural radiance and glow and/or a beautiful look of the skin. Most preferred the invention relates to the use of the compositions according to the invention for supporting a clear, pure appearance of the skin.

In another embodiment the invention relates to a cosmetic non therapeutic method of supporting healthy skin appearance and beauty, skin nourishment from inside the body via the blood stream (systemically), for supporting a clear, pure appearance of the skin, moisturizing the skin, supporting the skin barrier function, fostering hydration, and/or providing a general anti-aging effect, a natural radiance and glow and/or a beautiful appearance of the skin comprising the step of administering a composition containing an effective amount of
i) at least one component selected from the group consisting of polyunsaturated fatty acids and esters of polyunsaturated fatty acids such as ethyl esters, mono-, di- and triglyceridesters; and
ii) at least one component selected from the group consisting of carotenoids, to humans,
wherein the amount of the carotenoids is in the range of 25 to 80 % by weight, based on the total weight of the composition.

The amount of the carotenoids is preferably in the range of 30 to 75 % by weight, more preferably in the range of 40 to 60 % by weight, based on the total weight of the composition.

The term "an effective amount" as used herein refers to an amount necessary to obtain a physiological effect. The physiological effect may be achieved by one single dose or by repeated doses. The dosage administered may, of course, vary depending upon known factors, such as the physiological characteristics of the particular composition and its mode and route of administration; the age, health and weight of the recipient; the nature and extent of the symptoms; the kind of concurrent treatment; the frequency of treatment; and the effect desired and can be adjusted by a person skilled in the art. The necessary daily amounts may be applied in a single dosage or in multiple dosages.

The compositions according to the present invention can serve as supplements to food, feed and beverages, dietary supplements and as pharmaceutical formulations which may be solid - such as capsules or tablets - or liquid - such as solutions or suspensions. They may be administered in form of (fortified) food, dietary supplements, beverages, tablets, granules, capsules, pastes, food additives, or effervescent formulations. Further examples are cereals, cereal bars and dairy products (e. g. milk, buttermilk, soured milk, yogurt (drinks), curd, quark desserts and so on) containing the component(s) i) and ii) according to the invention.

The components according to the present invention may be administered together in one pharmaceutical form or separately in various pharmaceutical forms (such as in two different pharmaceutical forms). If two or more pharmaceutical forms are used, the pharmaceutical forms are preferably consumed by the human(s) at the same time.
"At the same time" as used herein means that the different pharmaceutical forms are orally consumed within a time period of 1 day, preferably within a time period of 1 h, more preferably within a time period of 5 minutes, even more preferably within a time period of 1 minute.

The pharmaceutical form(s) comprise the components according to the present invention and optionally a suitable excipient and/or carrier.

The term "pharmaceutical form" as used herein comprises gelcaps, capsules, powders, solid tablets (coated or non-coated), syrups, drink ampoules and sachets/pouches, preferably tablets or capsules such as hard (shell) gelatin capsules. Suitable excipient and/or carriers include maltodextrin, calcium carbonate, dicalcium phosphate, tricalcium phosphate, microcrystalline cellulose, dextrose, rice flour, magnesium stearate, stearic acid, croscarmellose sodium, sodium starch glycolate, crospovidone, sucrose, vegetable gums, lactose, methylcellulose, povidone, carboxymethylcellulose, corn starch, and the like (including mixtures thereof). Preferred carriers include calcium carbonate, magnesium stearate, maltodextrin, and mixtures thereof. The components according to the present invention are mixed with the excipient(s) and/or carrier(s) and formed into the desired form using conventional techniques. The tablet or capsule according to the present invention may be coated with an enteric coating that dissolves at a pH of about 6.0 to 7.0. A suitable enteric coating that dissolves in the small intestine but not in the stomach is cellulose acetate phthalate. Further details on techniques for formulation and administration may be found in the latest edition of Remington's Pharmaceutical Sciences (Maack Publishing Co., Easton, PA).

For example, if a sun (UV) protective effect is desired, it is advantageous to apply an oral composition according to the present invention that contains one or more active ingredients from the following table in combination with a topical composition that contains one or more active ingredients from the following table:

| **Prevention** | | **Immediate protection from acute sun damage** | |
|---|---|---|---|
| *Oral* | *Topical* | *Oral* e.g. as isotonic drink | *Topical* |
| Betacarotene Lutein | Ascorbyl Phosphate Vitamin E Dihydroxyaceton | Folic acid Vitamin B12 Vitamins A,C, E EGCG Lycopene | UV-filter substances (e.g. Parsols¹) Vitamin E |

| | | | |
|---|---|---|---|
| ¹The following Parsols are available from DSM Nutritional Products Ltd.: PARSOL® 1789: 1-(4-tert-butylphenyl)-3-(4-methoxyphenyl)-propane-1,3-dione PARSOL® 340: 2-cyano-3,3-diphenyl-acrylic acid 2-ethyl-hexyl ester PARSOL® 5000: (E)-rac-1,7,7-trimethyl-3-(4-methyl-benzylidene)-bicyclo-[2.2.1]-heptan-2-one PARSOL® EHS: 2-ethylhexyl salicylate PARSOL® HMS: 3,3,5-trimethylcyclohexyl salicylate PARSOL® HS: 2-phenyl-1H-benzimidazole-5-sulphonic acid PARSOL® MCX: 3-(4-methoxy-phenyl)-propionic acid 2-ethyl-hexyl ester; 2-ethylhexyl 3-(4-methoxyphenyl)-2-propenoate PARSOL® SLX: **CAS No**.: 207574-74-1 PARSOL® TX: Titanium Dioxide | | | |

If a sun (UV) protective effect is desired, the term "oral prevention" means intake of the active ingredient(s) months to days before sun exposure. "Topical prevention" means enrichment of the protective ingredient(s) by topical application months, days to hours before exposure to sun light.
"Immediate protection from acute sun damage" means application in minutes before and application during sun exposure. The term "protection" encompasses not only protection against sun burn (sun erythema), but also protection against damages through sunlight-induced oxidative stress and/or immune suppression and/or their consequences, i.e. photoaging.

| **Repair** | |
|---|---|
| *Oral* | *Topical* |
| Betacarotene | Panthenol |
| Coenzyme Q10 | Vitamin E |
| EGCG | Eicosapenta-5,8,11,14,17-enoic acid |
| Biotin | Coenzyme Q10 |
| Vitamin C | |
| Pantothenate | |
| Resveratrol | |
| B-Vitamins | |

The term "repair" as used in this context means application of the active ingredients after sun-light induced damage as described above has occurred.

If the prevention or treatment/reduction of cellulite is the desired effect, it is advantageous to apply an oral composition according to the present invention that contains one or more active ingredients from the following table in combination with a topical composition that contains one or more active ingredients from the following table:

| **Prevention of cellulite** | | **Treatment/reduction of cellulite** | |
|---|---|---|---|
| *Oral* | *Topical* | *Ora*/ | *Topical* |
| | e.g. massage cream or gel | | e.g. massage cream or gel |
| Betacarotene | Genistein | Betacarotene | Genistein |
| | Resveratrol | | Resveratrol |
| | Phytanic Acid | | Phytanic Acid |

If an anti-aging effect is desired, it is advantageous to apply an oral composition according to the present invention that contains one or more active ingredients from the following table in combination with a topical composition that contains one or more active ingredients from the following table:

| **Prevention** | | **Repair** | |
|---|---|---|---|
| *Oral* | *Topical* | *Oral* | *Topical* |
| Betacarotene | Colorless | Betacarotene | Resveratrol |
| Carotenoids (including colorless carotenoids) | carotenoids (phytoene, phytofluene) | Carotenoids (including colorless carotenoids phytoene, phytofluene) | Coenzyme Q10 |
| | | | Vitamin E |
| | | | Genistein |
| | | | Milk tripeptides |
| | | | Vitamins |
| | | | Panthenol |
| | | | Vitamin A |
| | | | (including Retinyl esters) |

If the prevention or treatment/reduction of sensitive and/or dry skin is the desired effect, it is advantageous to apply an oral composition according to the present invention that contains one or more active ingredients from the following table in combination with a topical composition that contains one or more active ingredients from the following table:

| **Prevention** | | **Acute anti-inflammation, soothing** | |
|---|---|---|---|
| *Oral* | *Topical* | *Oral* | *Topical* |
| Lycopene | Panthenol | Lycopene | Resveratrol |
| Beta-carotene | | Resveratrol | Hydroxytyrosol |
| Biotin | | Hydroxytyrosol | Panthenol |
| Hydroxytyrosol | | | |
| Vitamin E | | | |
| Resveratrol | | | |

The term "oral prevention" as used in this context means intake of the active ingredient(s) months to days before sensitive and/or dry skin occurs such as in winter season or after sun exposure. "Topical prevention" means enrichment of the protective ingredient(s) by topical application months, days to hours before sensitive and/or dry skin occurs.

"Acute anti-inflammation and soothing" as used in this context means application in minutes before and application when sensitive and/or dry skin is present. The term "protection" encompasses not only protection against sensitive and/or dry skin, but also normalization of sensitive and/or dry skin.

| **Repair** | |
|---|---|
| *Oral* | *Topical* |
| Betacarotene | Panthenol |
| Lutein | Vitamin E |
| Biotin | Phytanic acid |
| Hydroxytyrosol | |
| Vitamins E,C | |
| B-Vitamins | |

The term "repair" as used in this context means application of the active ingredients after sensitive and/or dry skin as described above has occurred.

The topical cosmetic compositions can also contain usual cosmetic adjuvants and additives, such as preservatives/ antioxidants, fatty substances/ oils, water, organic solvents, silicones, thickeners, softeners, emulsifiers, sunscreens, antifoaming agents, moisturizers, aesthetic components such as fragrances, surfactants, fillers, sequestering agents, anionic, cationic, nonionic or amphoteric polymers or mixtures thereof, propellants, acidifying or basifying agents, dyes, colorings/colorants, abrasives, absorbents, essential oils, skin sensates, astringents, antifoaming agents, pigments or nanopigments, e.g. those suited for providing a photoprotective effect by physically blocking out ultraviolet radiation, or any other ingredients usually formulated into cosmetic compositions. Such cosmetic ingredients commonly used in the skin care industry, which are suitable for use in the compositions of the present invention are e.g. described in the CTFA Cosmetic Ingredient Handbook, Second Edition (1992) without being limited thereto.

The necessary amounts of the cosmetic and dermatological adjuvants and additives can - based on the desired product - easily be chosen by a skilled person in this field and will be illustrated in the examples, without being limited hereto.

The usual cosmetic adjuvants and additives such as e.g. emulsifiers, thickeners, surface active ingredients and film formers can show synergistic effects which can be determined by the expert in the field with normal trials, or with the usual considerations regarding the formulation of cosmetic composition.

Which amount of the topical composition has to be applied, depends on the concentration of the active ingredient(s) in the product and the desired cosmetic effect(s). A typical "leave-on" composition like a skin care emulsion, for example, is usually applied in an amount of about 0.5 to about 2 mg per cm² skin. The applied amount is normally not critical, and the desired effect(s) may be achieved by using more of the composition, repeating the application of the composition and/or applying a composition which contains more of the active ingredient(s).

By "'leave-on' composition" as used herein a topical composition is meant which after having applied to the skin, is not removed intentionally. It is preferably left on the skin for a period of at least about 15 minutes, more preferably at least about 30 minutes, even more preferably at least about 1 hour, most preferably for at least several hours, e. g. up to about 12 hours.

According to the present invention, it is advantageous to (re-) apply the oral composition - and in the case of the personal skin care kit also the topical composition - on a continuous basis (repeatedly).

By "continuous (re-) application" as used herein is meant that the oral composition and - if applicable (see above) - the topical composition is/are applied at least once a day over an extended period during the subject's lifetime, preferably at least once a day for a period of about a week, more preferably at least once a day for a period of about one month, even more preferably at least once a day for about three months, even more preferably at least once a day for about six months, and most preferred at least once a day for about one year or more. Although benefits are normally obtained after shorter periods of use (e. g. after continuous application for weeks or months respectively), it is preferred that the application continues throughout the subject's lifetime to maintain the positive effects.

The following examples are provided to further illustrate the processes and compositions of the present invention. These examples are illustrative only and are not intended to limit the scope of the invention in any way.

### Comparative examples

An assay was performed to investigate the effect of selected compounds of this invention in an inflammation model with human skin keratinocytes.

In the assay, the human keratinocyte cell line HaCat was stimulated with the inflammatory cytokine TNFα and the release of Interleukin (IL)6 into the medium was analysed with a commercially available "Enzyme Linked Immunosorbent Assay" (ELISA).

### Method

### Experiment 1

HaCaT cells were cultivated under the conditions explained in detail in Wertz et al., Free Radical Biology & Medicine, Vol. 37, No. 5, pp. 654 - 670, 2004. Cells were pre-cultivated in medium containing with lycopene at a concentration of 1 µmol/L for 48h to give the cells sufficient time to accumulate lycopene. Lycopene was solubilized in a stock solution with Tetrahydrofuran (THF) and diluted into the medium such that the end concentration was 0.1 %THF. After the pre-incubation period, cells were stimulated with 10µg/ml TNFα for 5h, after which the medium was collected and frozen. Cells were harvested by trypsinisation and counted according to standard procedures. 116 levels in the medium were standardized to the number of cells in the wells and expressed as µg/10⁶ cells

### Result

**Table 2**

| Treatment | IL6 pg/ 10⁶ cells ± SD |
|---|---|
| Control (no TNFα) | 41.39 ± 7.76 |
| TNFα 10 µg/ml | 332.72 ± 6.22 |
| Lycopene 1µmol/L + TNFα 10 µg/ml µg/ml | 257.69* ± 17.47 |

| | |
|---|---|
| p<0.03, 22% reduction in response | |

This results show that lycopene as a representative of a carotenoid, is able to significantly reduce the reaction of human skin keratinocytes to a pro-inflammatory stimulus. This means that lycopene when ingested regularly, can reduce the predisposition of the reaction towards an irritating pro-inflammatory insult.

### Experiment 2

This experiment was performed to investigate whether two other carotenoid representatives, beta-carotene and beta-cryptoxanthin, are able to reduce the general base line production of inflammatory mediators of normal skin

| Treatment | IL6 pg/10⁶ cells ± SD |
|---|---|
| Control (no TNFα) | 293.3 |
| Beta-carotene 0.1 µmol/L | 315.86 |
| Beta-carotene 0.25 µmol/L | 225.15 |
| Beta-carotene 1.0µmol/L | 241.8 |
| Beta-carotene 3.0 µmol/L | 291.85 |
| Beta- cryptoxanthin 0.1 µmol/L | 264.76 |
| Beta- cryptoxanthin 0.25 µmol/L | 268.46 |
| Beta- cryptoxanthin 0.5 µmol/L | 286.77 |

This result shows that beta-carotene can reduce the base line level of skin cells to secrete the pro-inflammatory cytokine IL6 into their environment. Beta-carotene had a U-shaped dose relation ship, and only when it was present in a concentration between 0.25-1.0 µmol/L. this corresponds to human plasma concentration after moderate beta-carotene supplementation between 0.1-2.0 mg/day per person. Beta-cryptoxanthin had a moderate effect in the dose range of human known plasma concentration after low to medium high intakes between 0.050mg to 1mg/d.

### Formulation Examples:

### Example 1 "Ideal skin formula I" for oral consumption

Lecithin is dissolved in the ROPUFA® 75 n-3 '75'EE (ethylester, containing min. 75% n-3 fatty acid ethyl ester, stabilized with mixed tocopherol, ascorbyl palmitate and rosemary extracts; commercially available from DSM Nutritional Products AG, Kaiseraugst, Switzerland) and the beta-Carotene 30% FS, Lutein 20% FS (extracted from Tagetes erecta in Corn Oil and stabilized with DL-alpha-Tocopherol), redivivo™ (lycopene) 10% FS (in cornoil, stabilized with DL-alpha-Tocopherol) (commercially available from DSM Nutritional Products AG, Kaiseraugst, Switzerland) are added, vitamin C, Hidrox®, Biotin and CoEnzyme Q10 (as ALL-Q® 10% CWS/S commercially available from DSM Nutritional Products AG, Kaiseraugst, Switzerland) are mixed in a tumbler mixer for 5 minutes. This dry powder mix is dispersed in the oily mixture of ROPUFA®, carotene and lecithin and then encapsulated in capsules according to a commonly applied procedure.

| | |
|---|---|
| Beta-carotene | 3 mg (= 10 mg beta-Carotene 30 % FS) |
| Lycopene (redivio™) | 3 mg (= 30 mg redivivo™ (lycopene) 10 % FS) |
| Lutein | 3 mg (= 15 mg Lutein 20 % FS) |
| D/L-alpha-Tocopherol-acetate | 75 mg |
| Vitamin C | 75 mg (as fine powder) |
| ROPUFA® 75 n-3 '75'EE | 400 mg |
| Hidrox ® | 100 mg (provides 6 mg polyphenols and 2.5 mg hydroxytyrosol) |
| Biotin | 150 µg |
| CoEnzyme Q10 (ALL-Q®) | 5 mg (=50 mg ALL-Q® 10 % CWS/S) |
| Lecithin as mixed soybean phosphatides | 25 mg |

One capsule is taken per day together with a meal.

### Example 2 "Ideal skin formula II" for oral consumption

Lecithin is dissolved in the ROPUFA® 75 n-3 '75'EE (ethylester, containing min. 75% n-3 fatty acid ethyl ester, stabilized with mixed tocopherol, ascorbyl palmitate and rosemary extracts; commercially available from DSM Nutritional Products AG, Kaiseraugst, Switzerland) and the beta-Carotene 30% FS, Optisharp™ 20% FS (in cornoil stabilized with DL-alpha-Tocopherol) are added. TEAVIGO™, vitamin C, Hidrox®, Biotin and CoEnzyme Q10 (as ALL-Q® 10% CWS/S commercially available from DSM Nutritional Products AG, Kaiseraugst, Switzerland) are mixed in a tumbler mixer for 5 minutes. This dry powder mix is dispersed in the oily mixture of ROPUFA®, carotene and lecithin and then encapsulated in capsules according to a commonly applied procedure.

| | |
|---|---|
| Beta-carotene | 6 mg (= 20 mg beta-Carotene 30 % FS) |
| Zeaxanthin | 2.0 mg (= 10 mg Optisharp™ 20 % FS) |
| D/L-alpha-tocopherol-acetate | 300 mg |
| Vitamin C | 100 mg |
| TEAVIGO™ | 150 mg |
| ROPUFA® 75 n-3 '75'EE | 400 mg |
| Hidrox ® | 100 mg (provides 6 mg polyphenols and 2.5 mg hydroxytyrosol) |
| Biotin | 300 µg |
| CoEnzyme Q10 (ALL-Q®) | 20 mg (=200 mg ALL-Q® 10 % CWS/S) |
| Lecithin as mixed soybean phosphatides | 40 mg |

One capsule is taken per day together with a meal.

### Example 3 "A kit for oral consumption"

The liquid active ingredients beta-carotene, zeaxanthin, DL-alpha-tocoherol-acetate, and ROPUFA® 75 n-3 '75'EE can be provided within a capsule whereas the solid ingredients vitamin C, Hidrox®, Biotin and CoQ10 are provided in the form of a tablet. The tablet may be prepared with commonly known tabletting excipients such as dry binders e.g. microcrystalline cellulose, lactose, other carbohydrates or carbohydrate derivatives like starch, sorbitol, mannitol dextrins etc. A disintegrant like croscarmelllose or crospovidone may be added in an appropriate amount, as well as a lubricant like mg-stearate or a similar compound, a behenate polyethyleneglycol, or any other lubricant.

### Capsule

| | |
|---|---|
| Beta-carotene | 6 mg (= 20 mg beta-Carotene 30 % FS) |
| Zeaxanthin | 2.0 mg (= 10 mg Optisharp™ 20 % FS) |
| D/L-alpha-tocopherol-acetate | 300 mg |
| ROPUFA® 75 n-3 '75'EE | 400 mg |
| Biotin | 300 µg |
| Lecithin as mixed soybean phosphatides | 40 mg |

This oily mix is encapsulated in capsules according to a commonly applied procedure.

### Tablet

| | |
|---|---|
| Vitamin C | 110 mg (as Ascorbic Acid 90% Granulation) |
| TEAVIGO™ TG | 150 mg |
| Hidrox ® | 100 mg (provides 6 mg polyphenols and 2.5 mg hydroxytyrosol) |
| Biotin | 300 µg |
| CoEnzyme Q10 (ALL-Q®) | 20 mg (=200 mg ALL-Q® 10 % CWS/S) |
| Microcrystalline cellulose | 300 mg |
| Lactose | 300 mg |
| Crospovidone | 35 mg |
| Mg-Stearate | 24.7 mg |

Biotin and microcrystalline cellulose is mixed in a tumbler mixer for 10 min. Then, lactose is added and the composition mixed for 10 min again. Vitamin C, TEAVIGO™ TG, Hidrox®, CoQ10 and Crospovidone are combined with the other ingredients and mixed for 10 min. Finally, mg-stearate is added to the other components and mixed for another 2 min. The mixture is compressed to tablets.

In order to provide the kit for oral intake according to the invention, the tablet and the capsule are individually packed in separate containers and then packed together in a unitary form.

Per day one capsule and one tablet are taken at the same time together with a meal.

### Example 4 "A personal care kit"

A capsule as described in example 1 or 2 is used together with an anti-ageing cream. The anti-aging cream containing the ingredients indicated below can be prepared in a manner known per se.

### Anti-aging cream

| O/W emulsion | |
|---|---|
| **Ingredients** | **% (w/w)** |
| Glyceryl Myristate | 4.00 |
| Cetyl Alcohol | 2.00 |
| Steareth-2 | 2.00 |
| Steareth-21 | 2.00 |
| Isopropyl Myristate | 5.00 |
| Caprylic/Capric Triglyceride | 8.00 |
| BHT | 0.05 |
| Dimethicone | 2.00 |
| Phenoxyethanol & Methylparaben & Ethylparaben & Butylparaben & Propylparaben & Isobutylparaben | 0.80 |
| CoEnzyme Q10 (ALL-Q®) | 0.50 |
| Water | Ad 100 |
| Xanthan Gum | 0.50 |
| Disodium EDETA | 0.10 |
| Propylene Glycol | 4.00 |

In order to provide the personal care kit according to the invention, the capsule and the anti-ageing cream are individually packed in separate containers and then packed together in a unitary form.
The anti-ageing cream is applied once a day. One capsule is taken per day together with a meal.

## Claims

1. Composition for oral intake comprising
i) at least one component selected from the group consisting of polyunsaturated fatty acids and esters of polyunsaturated fatty acids - e.g. ethyl esters, mono-, di- and triglyceridesters; and
ii) at least one component selected from the group consisting of carotenoids, wherein the amount of carotenoids is in the range of 25 to 80 % by weight, based on the total weight of the composition.

2. Composition according to claim 1, wherein the component i) is essentially a mixture of eicosapentaenoic acid, docosahexaenoic acid and their esters.

3. Composition according to claim 2, wherein the esters are ethyl esters.

4. Composition according to one of the claims 1 to 3, wherein the carotenoids are selected from the group consisting of β-carotene, lutein, zeaxanthin, and lycopene and pharmaceutically acceptable derivatives thereof.

5. Non therapeutic use of a composition according to any of claims 1 to 4 for supporting a healthy skin appearance and beauty, for supporting skin nourishment from inside the body via the blood stream (systemically), for supporting a clear, pure appearance of the skin, for fostering hydration, for moisturizing the skin, for supporting the skin barrier function, and/or for providing a general anti-aging effect.

6. A composition according to any of claims 1 to 4 for use in the protection of skin against oxidative stress

7. A composition according to any of claims 1 to 4 for use in the protection of skin against UV-radiation.

8. Cosmetic non therapeutic method of supporting healthy skin appearance and beauty, skin nourishment from inside the body via the blood stream, for supporting a clear, pure appearance of the skin, moisturizing the skin, supporting the skin barrier function, fostering hydration, and/or providing a general anti-aging effect, in particular promoting a natural radiance and glow and/or a beautiful appearance of the skin comprising the step of administering a composition according to claim 1 containing an effective amount of
i) at least one component selected from the group consisting of polyunsaturated fatty acids and esters of polyunsaturated fatty acids such as ethyl esters, mono-, di- and triglyceridesters; and
ii) at least one component selected from the group consisting of carotenoids, to humans,
wherein the amount of carotenoids is in the range of 25 to 80 % by weight, based on the total weight of the composition.

## Patentansprüche

1. Zusammensetzung für die orale Aufnahme, die Folgendes umfasst:
i) mindestens eine Komponente, die aus der Gruppe bestehend aus mehrfach ungesättigten Fettsäuren und Estern von mehrfach ungesättigten Fettsäuren - z.B. Ethylestern, Mono-, Di- und Triglyceridestern - ausgewählt ist; und
ii)mindestens eine Komponente, die aus der Gruppe bestehend aus den Carotenoiden ausgewählt ist,
wobei die Menge an Carotenoiden im Bereich von 25 bis 80 Gew.-% in Bezug auf das Gesamtgewicht der Zusammensetzung liegt.

2. Zusammensetzung nach Anspruch 1, wobei es sich bei der Komponente i) im Wesentlichen um eine Mischung aus Eicosapentaensäure, Docosahexaensäure und ihre Ester handelt.

3. Zusammensetzung nach Anspruch 2, wobei es sich bei den Estern um Ethylester handelt.

4. Zusammensetzung nach einem der Ansprüche 1 bis 3, wobei die Carotenoide aus der Gruppe bestehend aus β-Carotin, Lutein, Zeaxanthin und Lycopen sowie pharmazeutisch unbedenklichen Derivaten davon ausgewählt sind.

5. Nichttherapeutische Verwendung einer Zusammensetzung nach einem der Ansprüche 1 bis 4 zur Förderung eines gesunden Hautbilds und einer gesunden Schönheit, zur Förderung der Ernährung der Haut aus dem Körperinneren über den Blutstrom (systemisch), zur Förderung eines klaren, reinen Hautbilds, zur Unterstützung der Hydratisierung, für das Spenden von Feuchtigkeit an die Haut, zur Förderung der Hautbarrierefunktion und/oder zur Bereitstellung einer allgemeinen Wirkung gegen das Altern.

6. Zusammensetzung nach einem der Ansprüche 1 bis 4 zur Verwendung beim Schützen der Haut gegen Oxidationsstress.

7. Zusammensetzung nach einem der Ansprüche 1 bis 4 zur Verwendung beim Schützen der Haut gegen UV-Strahlung.

8. Kosmetisches nichttherapeutisches Verfahren zur Förderung eines gesunden Hautbilds und einer gesunden Schönheit, zur Förderung der Ernährung der Haut aus dem Körperinneren über den Blutstrom, zur Förderung eines klaren, reinen Hautbilds, zur Unterstützung der Hydratisierung, für das Spenden von Feuchtigkeit an die Haut, zur Förderung der Hautbarrierefunktion und/oder zur Bereitstellung einer allgemeinen Wirkung gegen das Altern, insbesondere zur Förderung einer natürlich strahlenden und frischen Haut und/oder eines schönen Hautbilds, umfassend den Schritt des Verabreichens einer Zusammensetzung nach Anspruch 1, die eine wirksame Menge an
i) mindestens einer Komponente, die aus der Gruppe bestehend aus mehrfach ungesättigten Fettsäuren und Estern von mehrfach ungesättigten Fettsäuren - z.B. Ethylestern, Mono-, Di- und Triglyceridestern - ausgewählt ist; und ii)mindestens einer Komponente, die aus der Gruppe bestehend aus den Carotenoiden ausgewählt ist, enthält, an den Menschen,
wobei die Menge an Carotenoiden im Bereich von 25 bis 80 Gew.-% in Bezug auf das Gesamtgewicht der Zusammensetzung liegt.

## Revendications

1. Composition pour administration orale comprenant
i) au moins un composant choisi dans le groupe constitué d'acides gras polyinsaturés et d'esters d'acides gras polyinsaturés - par exemple des esters éthyliques, des esters mono-, di- et triglycéridiques ; et
ii) au moins un composant choisi dans le groupe constitué de caroténoïdes, la quantité de caroténoïdes étant dans la plage de 25 à 80 % en poids, sur la base du poids total de la composition.

2. Composition selon la revendication 1, dans laquelle le composant i) est essentiellement un mélange d'acide eicosapentaénoïque, d'acide docosahexaénoïque et leurs esters.

3. Composition selon la revendication 2, dans laquelle les esters sont des esters éthyliques.

4. Composition selon un des revendications 1 à 3, dans laquelle les caroténoïdes sont choisis dans le groupe constitué des β-carotène, lutéine, zéaxanthine, et lycopène et des dérivés pharmaceutiquement acceptables de ceux-ci.

5. Utilisation non thérapeutique d'une composition selon l'une quelconque des revendications 1 à 4 pour favoriser un aspect sain de la peau et la beauté, pour favoriser la nutrition de la peau depuis l'intérieur du corps via la circulation sanguine (systémique), pour favoriser un aspect clair et pur de la peau, pour stimuler l'hydratation, pour hydrater la peau, pour favoriser la fonction de barrière de la peau, et/ou pour produire un effet antivieillissement général.

6. Composition selon l'une quelconque des revendications 1 à 4 pour utilisation dans la protection de la peau contre le stress oxydatif.

7. Composition selon l'une quelconque des revendications 1 à 4 pour utilisation dans la protection de la peau contre le rayonnement UV.

8. Procédé cosmétique non thérapeutique pour favoriser un aspect sain de la peau et la beauté, la nutrition de la peau depuis l'intérieur du corps via la circulation sanguine, pour favoriser un aspect clair et pur de la peau, hydrater la peau, favoriser la fonction de barrière de la peau, et/ou produire un effet antivieillissement général, en particulier favoriser un rayonnement et un éclat naturels et/ou un aspect plaisant de la peau comprenant l'étape d'administration d'une composition selon la revendication 1 contenant une quantité efficace de
i) au moins un composant choisi dans le groupe constitué d'acides gras polyinsaturés et d'esters d'acides gras polyinsaturés - par exemple des esters éthyliques, des esters mono-, di- et triglycéridiques ; et
ii) au moins un composant choisi dans le groupe constitué de caroténoïdes,
à des humains,
la quantité de caroténoïdes étant dans la plage de 25 à 80 % en poids, sur la base du poids total de la composition.
